# EUROPEAN PATENT SPECIFICATION

(11) **EP 2 951 289 B1**
(45) Date of publication and mention of the grant of the patent: **02.10.2019**
(21) Application number: 14746794.8
(22) Date of filing: 30.01.2014
(51) Int. Cl.: C12N 5/071, A61L 27/56, A61L 27/38, A61L 27/36, A61L 27/54, C12N 5/073

(54) **PLACENTAL MEMBRANE PREPARATION AND METHODS OF MAKING AND USING SAME**
PLAZENTAMEMBRANPRÄPARAT UND VERFAHREN ZUR HERSTELLUNG UND VERWENDUNG DAVON
PRÉPARATION DE MEMBRANE PLACENTAIRE ET PROCÉDÉS DE FABRICATION ET D'UTILISATION ASSOCIÉS

(30) Priority: 30.01.2013 US 201313754716; 30.01.2013 US 201313754742
(43) Date of publication of application: 09.12.2015
(73) Proprietor: Nutech Medical, Inc., Birmingham, AL 35216 (US)
(72) Inventor: TABET, Samuel, K., Albuquerque, NM 87104 (US); YAGER, Gregory, J., Mount Olive, AL 35117 (US); WALTHALL, Howard, P., Birmingham, AL 35213 (US)
(74) Representative: Elsy, David
(86) International application number: PCT/US2014/013893
(87) International publication number: WO 2014/120946

(56) References cited:
- EP-A1- 1 944 045
- WO-A2-2013/049052
- US-A- 6 152 142
- US-A1- 2003 187 515
- US-A1- 2005 074 476
- US-A1- 2010 104 539
- US-A1- 2011 206 776
- US-A1- 2013 138 222
- ZHOU, S. ET AL.: 'Demineralized bone promotes chondrocyte or osteoblast differentiation of human marrow stromal cells cultured in collagen sponges' CELL TISSUE BANK vol. 6, no. 1, 2005, pages 33 - 44, XP019234045
- THITISET, T. ET AL.: 'Development of collagen/demineralized bone powder scaffolds and periosteum-derived cells for bone tissue engineering application' INT. J. MOL. SCI. vol. 14, 1, 21 January 2013, pages 2056 - 2071, XP055214774

## Description

### Technical Field

The present invention is directed to a placental membrane preparation. More particularly, the present invention is directed to a placental membrane preparation and methods of making and using same.

### Background of Invention

The placenta surrounds a fetus during gestation and is composed of, among other tissues, an inner amniotic layer that faces the fetus and a generally-inelastic outer shell, or chorion. The placenta anchors the fetus to the uterine wall, allowing nutrient uptake, waste elimination, and gas exchange to occur via the mother's blood supply. Additionally, the placenta protects the fetus from an immune response from the mother. From the placenta, an intact placental membrane comprising the amnion and chorion layers can be separated from the other tissues.

Clinicians have used intact placental membrane, comprising an amnion and a chorion layer, in medical procedures since as early as 1910 [Davis, J.S., John Hopkins Med. J. 15, 307 (1910)]. The amniotic membrane, when separated from the intact placental membrane, may also be used for its beneficial clinical properties [Niknejad H, et al. Eur Cell Mater 15, 88-99 (2008)]. Certain characteristics of the placental membrane make it attractive for use by the medical community. These characteristics include, but are not limited to, its anti-adhesive, anti-microbial, and anti-inflammatory properties; wound protection; ability to induce epithelialization; and pain reduction. [Mermet I, et al. Wound Repair and Regeneration, 15:459 (2007)].

Other uses for placental membrane include its use for scaffolding or providing structure for the regrowth of cells and tissue. An important advantage of placental membrane in scaffolding is that the amnion contains an epithelial layer. The epithelial cells derived from this layer are multipotent cells, allowing the cells to multiply and differentiate into cells of other types. Multipotent cells are also contained within the body of the amniotic membrane. Additionally, the amniotic membrane contains various growth and trophic factors, such as epidermal, insulin-like, and fibroblast growth factors, as well as high concentrations of hyaluronic acid, that may be beneficial to prevent scarring and inflammation and to support healing. Thus, placental membrane offers a wide variety of beneficial medical uses.

Cell-based therapies have considerable potential for the repair and regeneration of tissues. The addition of a scaffold to these cell-based therapies has yielded improved outcomes [Krishnamurithy G, et al. J Biomed Mater Res Part A 99A, 500-506 (2011)]. Ideally, the material used for the scaffold will be biocompatible such that it provokes little to no immune response, biodegrades, and is available in sufficient quantities to be practical. Although the placental membrane has long been identified as a materially potentially filling this role in the clinic, efforts have been limited to *in vitro* studies, impractical *in vivo* techniques, or have yielded less than optimal outcomes. Furthermore, the conditions under which the scaffold is used may have a dramatic effect on the therapeutic efficacy.

Multiple studies exist expounding on the potential uses of human amniotic cells in various platforms for tissue repair. It has been proven that amniotic cells are multipotent in nature and can be influenced to produce various cell lines including chondrocytes. Further, it has been shown in the lab that demineralized bone can influence multipotent cells to produce both chondrocyte and osteoblast type cells.

Articular cartilage, located on the articular ends of bones at joints throughout the body, is composed of hyaline cartilage and contains relatively few chondrocytes that are embedded in extracellular matrix materials, such as type II collagen and proteoglycan [Moriya T, et al. J Orthop Sci 12, 265-273 (2007)]. Articular cartilage has a limited ability to self-repair, in part due to the avascular characteristics of the cartilage, which poses a significant challenge to treating joint injuries or diseases. The repair of cartilage defects in humans can therefore be a difficult endeavor, and multiple options exist for the surgeon to approach this topic. The surgeon may choose to influence the defect with microfracture of abrasion techniques to stimulate bleeding and a resulting fibrocartilage patch in which to fill the defect. There are also options available that allow for the filling of the defect with chondrocytes of variable sources, both of autograft and allograft origin.

However, current treatments, including cell-based therapies, have resulted in the generation of undesirable fibrocartilaginous tissue rather than hyaline cartilage [Diaz-Prado SM, et al. BIOMEDICAL ENGINEERING, TRENDS, RESEARCH, AND TECHNOLOGIES, pp. 193-216 (2011)]. As such, there remains a significant clinical need for therapies capable of repairing damaged articular cartilage that are capable of regenerating hyaline cartilage.

### Summary of the Disclosure

The present invention is directed to a placental membrane preparation and methods of making and using same. The invention is directed to a placental membrane preparation including demineralized bone powder and a placental membrane sheet as defined in claim 1. The chorion layer may be excluded from the placental membrane sheet. DBP may be applied to a stromal layer of the placental membrane sheet. The DBP may be reconstituted. The placental membrane sheet is folded to form an implantable unit. A portion of the amnion side of the implantable unit may be exposed. The amnion side of the placental membrane sheet may be covered with a layer of placental epithelial cells. The chorion side of the placental membrane sheet may include a stromal surface layer. The placental membrane preparation may or may not include chondrocytes. The chondrocytes may be derived from autologous or allograft chondrocytes or multipotent cells originating from the placental membrane sheet. The placental membrane sheet may include viable placental membrane cells.

In another embodiment, the invention is directed to a method of making a placental membrane preparation including applying a demineralized bone powder to a placental membrane sheet as defined in claim 11. The DBP, which may be reconstituted, may be applied to a stromal layer of the placental membrane sheet. The chorion layer may be removed prior to applying DBP. The placental membrane sheet may be essentially free of chondrocytes prior to implantation into a patient since chondrogenic differentiation of the placental membrane cells may occur *in vivo.* Chondrogenic differentiation may be induced in a plurality of multipotent cells of the placental membrane sheet. This differentiation may be induced in an essentially oxygen-free environment or in the absence of blood components. The placental membrane sheet may be dried, rehydrated, and DBP may be applied before, during, or after rehydration of the placental membrane sheet. An implantable unit is formed by folding the placental membrane sheet.

The scope of the invention is defined by the claims. Any references in the description to methods of treatment refer to the compounds, pharmaceutical compositions and medicaments of the present invention for use in a method for treatment of the human (or animal) body by therapy (or for diagnosis). In another embodiment, the invention is directed to a method of generating cartilage *in vivo* in a skeletal joint, the method including implanting a placental membrane preparation, as described herein, into the skeletal joint wherein the placental membrane nrenaration comprises a placental membrane sheet and a demineralized bone powder applied thereto. The cartilage that is generated may comprise, in whole or part, hyaline articular cartilage. The method of generating cartilage *in vivo* may comprise placing a patch over the preparation and suturing the patch to the skeletal joint.

In another embodiment, the invention is directed to a method of generating cartilage *in vivo* in a skeletal joint, the method including removing a diseased cartilage portion from a cartilage body of the skeletal joint, wherein the leakage of blood caused by removing the diseased cartilage portion is minimized, and essentially all of the blood from the skeletal joint is removed prior to implantation of a placental membrane sheet into the skeletal joint. The diseased cartilage may be removed in a manner that leaves sustainably all of the healthy cartilage intact. Removing the diseased cartilage portion may expose an underlying bone. The underlying bone may be intact. The underlying bone may be leaking blood, which, along with any blood clots, may be removed. A layer of bone wax may be applied to any void caused by the removal of diseased or damaged cartilage from the skeletal joint. A placental membrane sheet, with or without DBP, may be implanted into a void formed in the cartilage body caused by removal of diseased cartilage.

In another embodiment, the invention is directed to a method of generating cartilage *in vivo* in a skeletal joint, the method including providing an insert including a placental membrane sheet and a collagen matrix that may contain one or more growth factors, and implanting the insert into the skeletal joint. The growth factor may be DBP. The implant may be inserted without disturbing any calcified cartilage therein. The insert may have an epithelial layer such that the epithelial layer makes up at least 90% of the insert's exterior. The insert may be positioned such that the insert includes an epithelial layer having a first portion facing toward a skeletal joint bone and a second portion facing away from the skeletal joint bone. A layer of bone wax may be applied to any void caused by the removal of diseased or damaged cartilage from the skeletal joint. The diseased or damaged cartilage may be removed without perforating subchondral bone. An epithelial layer of the placental membrane may be applied to the walls of the void.

A further understanding of the nature and advantages of the present invention will be realized by reference to the remaining portions of the specification and the drawings of the present application.

### Brief Description of the Drawings

FIG. 1 is a top plan view of a placental membrane sheet in accordance with a preferred embodiment of the present invention.
FIG. 2 is a sectional view of a placental membrane sheet in accordance with a preferred embodiment of the present invention.
FIG. 3 is a sectional view of a placental membrane sheet in accordance with a preferred embodiment of the present invention.
FIG. 4 is a sectional view of a placental membrane implant prepared using the placental membrane sheet of FIG. 2.
FIG. 5 is a sectional view of a placental membrane implant prepared using the placental membrane sheet of FIG. 3.
FIG. 6 is a sectional view of an articular joint depicting a void created in accordance with a preferred embodiment of the present invention.
FIG. 7 is sectional view of the articular joint of FIG. 6 including the placental membrane implant of FIG. 4.
FIG. 8 is a sectional view of the articular joint of FIG. 6 including the placental membrane implant of FIG. 3.
FIG. 9 is a sectional view of the articular joint of FIG. 6 including multiple unfolded placental membrane implantable units.
FIG. 10 depicts a cartilage defect in a sheep knee.
FIG. 11 depicts a placental membrane sheet.
FIG. 12 depicts the preparation of an implantable placental membrane unit.
FIG. 13 depicts implantation of an implantable placental membrane unit into a sheep knee cartilage defect.
FIG. 14 is a low power histological view of a hematoxylin and eosin (HE) stained cartilage defect in a control sheep.
FIG. 15 is a high power histological view of a HE stained cartilage defect in a control sheep.
FIG. 16 is a low power histological view of a HE stained cartilage defect in a normal sheep.
FIG. 17 is a low power histological view of a trichrome stained cartilage defect in a normal sheep.
FIG. 18 is a high power histological view of an HE stained cartilage defect in a test sheep.
FIG. 19 is a low power histological view of an HE stained cartilage defect in a test sheep.
FIG. 20 is a high power histological view of an trichrome stained cartilage defect in a test sheep.
FIG. 21 is a low power histological view of an trichrome stained cartilage defect in a test sheep.

### Description of the Preferred Embodiments

Before the present compositions, articles, devices, and/or methods are disclosed and described, it is to be understood that they are not limited to specific methods unless otherwise specified, or to particular reagents unless otherwise specified, and as such may vary. It is also to be understood that the terminology as used herein is used only for the purpose of describing particular embodiments and is not intended to be limiting.

### A. Definitions

In this specification, and in the claims that follow, reference is made to a number of terms that shall be defined to have the following meanings:
As used herein, the singular forms "a," "an," and "the" include plural referents unless the context clearly indicates otherwise. Thus, for example, reference to "a pharmaceutical carrier" includes mixtures of two or more such carriers, and the like.

As used herein, ranges can be expressed as from "about" one particular value, and/or to "about" another particular value. When such a range is expressed, an embodiment includes from the one particular value and/or to the other particular value. Similarly, when values are expressed as approximations, by the use of "about," it will be understood that the particular value forms another embodiment. It will be understood that the endpoints of each of the ranges are significant both in relation to the other endnoint and independently of the other endpoint. It will also be also understood that there are a number of values disclosed herein, and that each value is also disclosed herein as "about" that particular value in addition to the value itself. For example, if the value "50" is disclosed, then "about 50" is also disclosed.

As used herein, the terms "optional" or "optionally" mean that the subsequently described event or circumstance may or may not occur, and that the description includes instances where said event or circumstance occurs and instances where it does not occur.

As used herein, the phrase "essentially oxygen-free environment" refers to an environment in which the free oxygen content is less than that of normal air, for example in an within articular cartilage. The term "free oxygen" refers to oxygen that is not combined with one or more other elements. Similarly, the phrase "essentially free of chondrocytes" refers to a composition which does not natively contain chondrocytes, and to which no extraneous chondrocytes have been added. For example, a placental membrane sheet that is essentially free of chondrocytes is a placental membrane sheet in which chondrocytes are not seeded onto the membrane.

As used herein, the phrase "essentially all chondrocytes" refers to a majority of the chondrocytes. Preferably, this refers to the maximum percentage of chondrocytes that can be reasonably attained by one of skill in the art. For example, where essentially all of the chondrocytes are derived from a particular source, other sources of chondrocytes may be excluded, inhibited, or reduced.

As used herein, the phrase "substantially all" refers to the maximum amount reasonably attainable by one skilled in the art. For example, removing diseased cartilage from a knee joint that leaves "sustainably all healthy portions of the cartilage body" indicates the removal of diseased cartilage that removes as little healthy cartilage as reasonably possible by one skilled in the art, such as a surgeon.

As used herein, the phrase "diseased cartilage" refers to cartilage that is damaged, degenerating, inflamed, necrotic, or otherwise showing symptoms thereof, such as pain, swelling, stiffness, and restraint of movement. Diseased cartilage may be diagnosed in several ways including, but not limited to, x-ray analysis, MRI analysis, or arthroscopy.

As used herein, the phrase "calcified cartilage" refers to the zone of cartilage that connects articular cartilage to the underlying subchondral bone.

As used herein, the phrase "bone wax" refers to a hemostatic material used to control bleeding from the surface of bone. For example, bone wax may be comprised of beeswax. In another example, bone wax comprises beeswax and one or more softening agents, such as paraffin. In another example, bone wax may comprise other inert hemostatic compounds, such as alkylene oxide copolymers.

As used herein, the phrases "placental membrane sheet" or "placental membrane" refer to one or more layers of the placental membrane. For example, placental membrane sheet may refer to a placental membrane comprising both the amniotic and chorionic layers. In another example, placental membrane sheet may refer to a placental membrane in which the chorion has been removed. In another example, placental membrane sheet may refer to a placental membrane in which the epithelial layer has been removed.

As used herein, the phrase "implantable unit" or "implant" refers to a mechanical configuration of a composition, comprising a placental membrane sheet, such that the composition is capable of insertion into or covering a surgical site. For example, an implantable unit may be a composition, comprising a placental membrane sheet, such that the composition is folded to permit insertion of the composition into a skeletal joint, such as the knee or shoulder, during surgery. In another example, an implantable unit may be a composition, comprising a placental membrane sheet, such that the composition is folded to permit covering a portion or an entirety of a skeletal joint during surgery.

As used herein, the term "patch" refers to a biocompatible composition. For example, a patch may comprise a placental membrane sheet. In another example, a natch comnrises a portion of amnion.

As used herein, the phrase "subchondral bone" refers to bone underlying cartilage. Subchondral bone may or may not be attached to the cartilage.

As used herein, the phrase "skeletal joint bone" refers to a bone in contact, or associated, with a skeletal joint. For example, a skeletal joint bone associated with the knee joint may include the femur.

As used herein, the phrase "demineralized bone powder" or "DBP" refers to a demineralized bone composition comprised of bone particles. DBP compositions may comprise fine powders, coarse grains, or even chips and are well known to those skilled in the art [Zhou S, et al. Cell Tissue Bank 6, 33-44 (2005)].

As used herein, the phrase "chondrogenic differentiation" refers to the differentiation of one cell type into a chondrocyte or chondrocyte-like cell. For example, mesenchymal stem cells may undergo chondrogenic differentiation such that they differentiate into chondrocytes.

As used herein, the phrase "reconstituted DBP" refers to DBP to which a compatible solvent has been added.

As used herein, the terms "treatment" or "treating" include any desirable effect on the symptoms or pathology of a disease or condition, and may include even minimal reductions in one or more measurable markers of the disease or condition being treated. "Treatment" does not necessarily indicate complete eradication or cure of the disease or condition, or associated symptoms thereof. The subject receiving this treatment is any animal in need, including primates, in particular humans, and other mammals including, but not limited to, equines, cattle, swine, and sheep; and poultry and pets in general.

### B. Methods of Making Placental Membrane Preparation

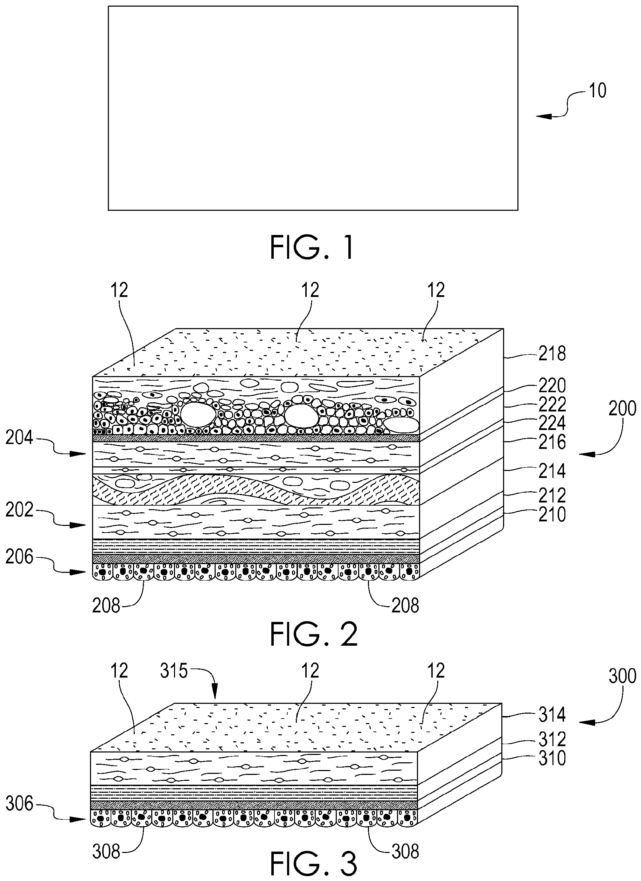

FIGS. 1 depicts a general shape of placental membrane sheet 100 in accordance with a preferred embodiment of the present invention. Placental membrane sheet 100 is of the type of membrane that is commonly used by clinicians in wound healing, cell regeneration, and tissue grafting applications.

FIG. 2 depicts an intact placental membrane sheet 200 including an amount of DBP 12 in accordance with a preferred embodiment of the present invention. Intact placental membrane 200 includes an amnion 202 and a chorion 204. Amnion 202 includes an epithelium 206 composed of a monolayer of epithelial cells 208, a basement membrane 210, a compact stromal layer 212, fibroblast layer 214 containing mesenchymal cells and a spongy layer 216. Chorion 204 includes a trophoblast layer 218, a basement membrane 220, a reticular layer 222 and a cellular layer 224. As illustrated, DBP 12 is applied to trophoblast layer 218.

FIG. 3 depicts a placental membrane sheet 300 including an amount of DBP 12 in accordance with another preferred embodiment of the present invention. Placental membrane 300 includes an amnion with the chorion removed, an amnion side 313 and a chorion side 315. Amnion includes an epithelium 306 composed of a monolayer of epithelial cells 308, a basement membrane 310, a compact stromal layer 312, and a fibroblast layer 314 containing mesenchymal cells. As illustrated, DBP 12 is applied to fibroblast layer 314. However, it is anticipated that removal of the chorion from placental membrane 300 will expose stromal layer 312 so that DBP 12 may be applied directly to stromal layer 312.

### 1. Initial Treatment and Removal of Particular Layers of the Placental Membrane

Placental membrane sheets 100, 200 and 300, depicted in FIGS. 1-3, and similar placental membrane materials may be produced from placentas collected from consenting donors in accordance with the Current Good Tissue Practice guidelines promulgated by the U.S. Food and Drug Administration.

In particular, soon after the birth of a human infant via a Cesarean section delivery, the intact placenta is retrieved, and the placental membrane is dissected from the placenta. Afterwards, the placental membrane is cleaned of residual blood, placed in a bath of sterile solution, stored on ice and shipped for processing. Once received by the processor, the placental membrane is rinsed to remove any remaining blood clots, and if desired, rinsed further in an antibiotic rinse [Diaz-Prado SM, et al. Cell Tissue Bank 11, 183-195 (2010)].

The antibiotic rinse may include, but is not limited to, the antibiotics: amikacin, aminoglycosides, amoxicillin, ampicillin, ansamycins, arsphenamine, azithromycin, azlocillin, aztreonam, bacitracin, capreomycin, carbacephem, carbapenems, carbenicillin, cefaclor, cefadroxil, cefalexin, cefalotin, cefamandole, cefazolin, cefdinir, cefditoren, cefepime, cefixime, cefoperazone, cefotaxime, cefoxitin, cefpodoxime, cefprozil, ceftaroline fosamil, ceftazidime, ceftibuten, ceftizoxime, ceftobiprole, ceftriaxone, cefuroxime, chloramphenicol, ciprofloxacin, clarithromycin, clindamycin, clofazimine, cloxacillin, colistin, cycloserine, dapsone, daptomycin, demeclocycline, dicloxacillin, dirithromycin, doripenem, doxycycline, enoxacin, ertapenem, erythromycin, ethambutol, ethionamide, flucloxacillin, fosfomycin, furazolidone, fusidic acid, gatifloxacin, geldanamycin, gentamicin, glycopeptides, grepafloxacin, herbimycin, imipenem or cilastatin, isoniazid, kanamycin, levofloxacin, lincomycin, lincosamides, linezolid, lipopeptide, lomefloxacin, loracarbef, macrolides, mafenide, meropenem, methicillin, metronidazole, mezlocillin, minocycline, monobactams, moxifloxacin, mupirocin, nafcillin, nalidixic acid, neomycin, netilmicin, nitrofurans, nitrofurantoin, norfloxacin, ofloxacin, oxacillin, oxytetracycline, paromomycin, penicillin G, penicillin V, piperacillin, platensimycin, polymyxin B, pyrazinamide, quinolones, quinupristin/dalfopristin, rifabutin, rifampicin or rifampin, rifapentine, rifaximin, roxithromycin, silver sulfadiazine, sparfloxacin, spectinomycin, spiramycin, streptomycin, sulfacetamide, sulfadiazine, sulfamethizole, sulfamethoxazole, sulfanilamide, sulfasalazine, sulfisoxazole, sulfonamidochrysoidine, teicoplanin, telavancin, telithromycin, temafloxacin, temocillin, tetracycline, thiamphenicol, ticarcillin, tigecycline, tinidazole, tobramycin, trimethoprim, trimethoprim-sulfamethoxazole (co-trimoxazole) (TMP-SMX), troleandomycin, trovafloxacin, or vancomycin.

The antibiotic rinse may also include, but is not limited to, the antimycotics: abafungin, albaconazole, amorolfin, amphotericin B, anidulafungin, bifonazole, butenafine, butoconazole, caspofungin, clotrimazole, econazole, fenticonazole, fluconazole, isavuconazole, isoconazole, itraconazole, ketoconazole, micafungin, miconazole, naftifine, nystatin, omoconazole, oxiconazole, posaconazole, ravuconazole, sertaconazole, sulconazole, terbinafine, terconazole, tioconazole, voriconazole, or other agents or compounds with one or more anti-fungal characteristics.

The placental membrane may be processed to remove one or more particular layers of the membrane. The chorion may be removed from the placental membrane by mechanical means well-known to those skilled in the art. The chorion may be removed, for example, by carefully peeling the chorion from the remainder of the placental membrane using blunt dissection [Jin CZ, et al. Tiss Eng 13, 693-702 (2007)]. Removal of the epithelial layer from the placental membrane may be achieved using several methods well-known to those skilled in the art. The epithelial layer may be removed by, for example, using trypsin to induce necrosis in the epithelial cells [Diaz-Prado SM, et al. Cell Tissue Bank 11, 183-195 (2010)]. Removal of the epithelial layer may comprise, for example, treatment with 0.1% trypsin-ethylenediaminetetraacetic acid (EDTA) solution at 37C for 15 minutes followed by physical removal using a cell scraper [Jin CZ, et al. Tiss Eng 13, 693-702 (2007)].

The placental membrane may then be stored in packs containing a sterile solution, air dried, or freeze dried. Both air drying and freeze drying are well known to those skilled in the art [Boo L, et al. Malay Orthop J 3, 16-23 (2009)]. The placental membrane may be air dried, for example, by spreading the membrane under a laminar flow or bio-safety hood, or like environment wherein the possibility of contamination is reduced, until dry. Typically, the placental membrane may be air dried overnight, typically for approximately 6 hours or more or preferably for 12 hours or more. The placental membrane may be freeze dried, for example, by placing the stretched placental membrane into a plastic bag in a freeze drier until dry. The placental membrane may be frozen prior to transfer to a freeze drier. Typically, the placental membrane may be freeze dried for approximately 6 hours or more or preferably for 12 hours or more. If the placental membrane is stored in a sterile solution, it may be stored at room temperature, cold stored at refrigerator temperatures, or cryopreserved at a temperature bellowing the freezing temperature of the solution.

The placental membrane preparation may be sterilized, typically using irradiation, as is well-known to those skilled in the art. Approximately 25 kGy gamma irradiation, for example, may be used for sterilization of the placental membrane preparation [Krishnamurithy G, et al. J Biomed Mater Res Part A 99A, 500-506 (2011)]. The placental membrane may be rehydrated using, for example, a sterile buffered saline solution [U.S. Patent No. 20030187515].

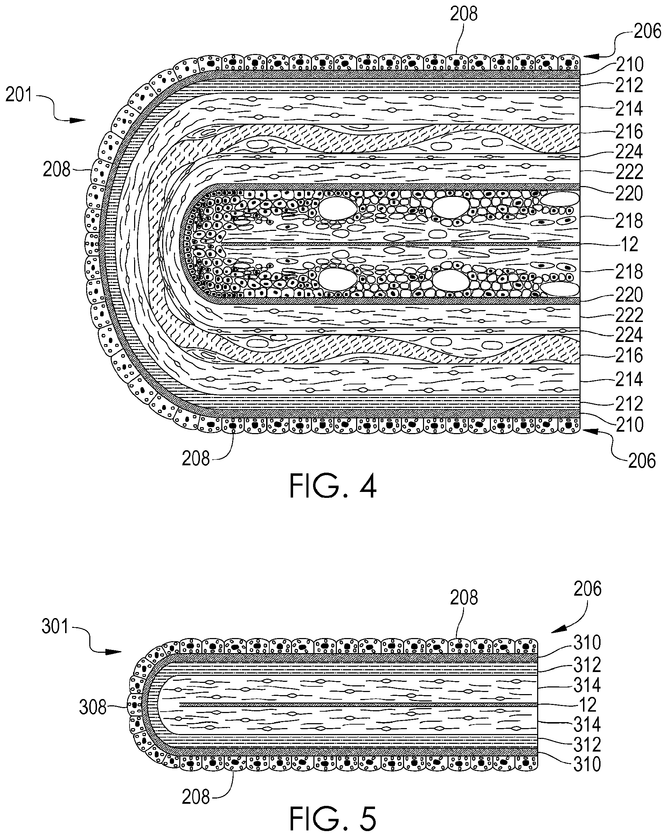

Referring to FIGS. 4 and 5, placental membrane sheets 200 and 300 may be folded to form implantable units 201 and 301, respectively. One or more folds may be created in the placental membrane sheet to permit the placental membrane preparation to fit or be in the proper orientation at the target site *in vivo.* The placental membrane sheet is in a manner that at least about 90% of the exterior surface of the implantable unit exposes an epithelial layer that may then be inserted such that the epithelial layer is in direct contact with cartilage. Folds may also be created in the placental membrane sheet to expose a particular side or layer, such as amniotic or epithelial, of the placental membrane preparation.

The placental membrane sheet may be combined with a collagen matrix. A collagen matrix is a three-dimensional scaffold comprising one or more forms of collagen including, but not limited to, for example, type I collagen, type II collagen, and type IV collagen. In addition, the collagen matrix may include one or more growth factors including, but not limited to, for example, TGF-β. A collagen matrix may be prepared using a variety of methodologies well-known to those skilled in the art. For example, a porous collagen matrix may be created by using pepsin-digested bovine collagen that is neutralized with 1 M HEPES at pH 7.4, 1 M NaH-CO₃, poured into a mold, frozen, lyophilized, and then irradiated [Zhou S, et al. Cell Tissue Bank 6, 33-44 (2005)].

### 2. Chondrocyte Differentiation

*In vitro* laboratory studies have indicated that chondrogenic differentiation may be induced in multiple cell types by the application of DBP under the appropriate culture conditions. Chondrogenesis may be induced in, for example, human dermal fibroblasts or human marrow stromal cells (hMSCs) using DBP and chondrogenic medium in combination with a collagen sponge system [Zhou S, et al. Cell Tissue Bank 6, 33-44 (2005)]. However, in this system under other culture conditions differentiation into osteoblasts may also result from the application of DBP. In fact, demineralized bone products are currently in surgical use primarily for the stimulation of bone growth. In the context of a diseased joint, the growth of bone spurs or other ectopic bone growth is not desirable and would likely cause a worsening of the condition of the joint

The differentiation of multiple cells types, such as mesenchymal stem cells, into chondrocytes may also be significantly affected by the presence or absence of growth factors. In the formation of cartilage, for example, TGF-β has been shown to have a substantial role [Hildner F, et al. J Tissue Eng Regen Med 5, e36-e51 (2011)]. In the placental membrane, the epithelial layer is a source of several growth factors including, but not limited to, EGF, KGF, HGF, and bFGF [Niknejad H, et al. Eur Cell Mater 15, 88-99 (2008)]. The epithelial layer also includes cytokines, such as activin, NGF, noggin, and TNF-α, that may play a role in cell differentiation. If the epithelial layer is removed from the placental membrane, as described herein, it is possible to seed the resulting membrane with epithelial or mesenchymal stem cells from the particular patient to avoid inducing or mitigating an immune response that may otherwise occur with allogeneic cells. Alternatively, exogenous growth factors may be introduced to induce the differentiation of placental membrane cells or of cells seeded thereon.

For the placental membrane preparation, chondrocytes may be derived from particular cell types. Chondrocytes may be derived from particular cell types by, for example, isolating that cell type, culturing, and differentiating either *in vitro* or *in vivo.* Particular cell types may be isolated using a variety of techniques well-known to those skilled in the art including, but not limited to, adherence to tissue culture plates or separation via cell sorting devices (e.g. autoMACS® Pro Separator, Miltenyi Biotec). The purity of a particular cell type within the pool of isolated cells may be tested using, for example, flow cytometry by which a distinctive set of surface or intracellular markers may be analyzed to ensure purity. The purity of a particular cell type within the pool of isolated cells may be tested using, for example, morphological analysis via microscopy. Both flow cytometry and morphological analysis via microscopy are well-known to those skilled in the art.

Cell viability may be assessed using a variety of techniques well-known to those skilled in the art including, but not limited to, flow cytometry or morphological analysis via microscopy. Flow cytometry using, for example, antibodies specific for annexin-V and propidium iodide will indicate cells that are apoptotic or necrotic, respectively.

The placental membrane sheet may be seeded using a variety of cell types. Isolated autologous or allograft chondrocytes, for example, may be seeded onto the placental membrane sheet *in vitro.* Similarly, other cell types, such as mesenchymal stem cells, may be seeded and subsequently differentiated into chondrocytes, either *in vitro* or *in vivo,* as described herein. In addition, placental membrane cells may be differentiated into chondrocytes. Unexpectedly, the in vitro application of placental membrane and DBM under the conditions described herein results in the growth and differentiation of chondrocyte-like cells, without differentiation of cells into osteoblasts resulting in ectopic bone growth.

### C. Uses of the Placental Membrane Preparation

The embodiments of the placental membrane preparation, described herein, may be used to regenerate damaged or defective tissue. Preferably, the embodiments of the placental membrane preparation, described herein, may be used to regenerate hyaline articular cartilage *in vivo,* with essentially no fibrocartilage generation and without the growth of bone spurs or other ectopic bone growth. The compositions and methods pertaining to the placental membrane preparation may be used in a number of clinical conditions including, but not limited to, chondral defects, osteoarthritis, traumatic injury, such as rotational or compaction injuries, osteochondritis dessicans, pathological injury, age-related degeneration, and other defects affecting skeletal joints, in particular cartilage.

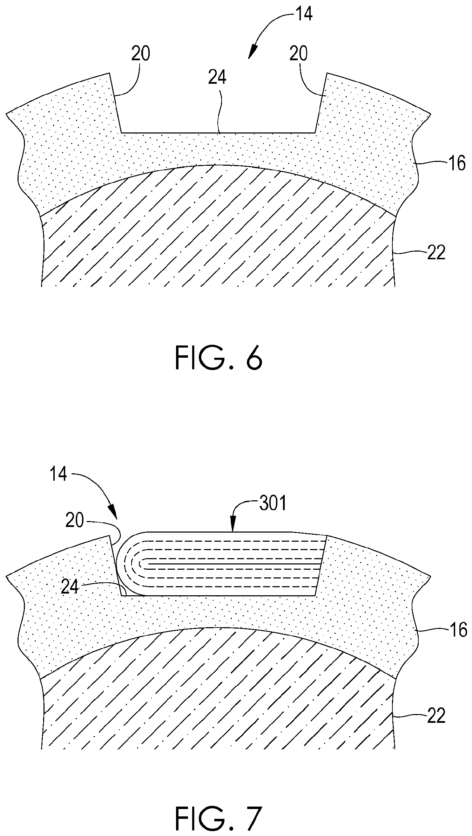

Referring to FIGS. 6 and 7, a placental membrane preparation such as implantable unit 301 may be implanted into a particular site *in vivo,* such as a skeletal joint, using surgical techniques well-known to those skilled in the art. The placental membrane preparation may be implanted, for example, into a void 14 in the articular cartilage 16 in a skeletal joint for the purpose of regenerating hyaline articular cartilage within void 14. Void 14 includes a sidewall 20 of cartilage, an upper opening and a bottom 24. Preferably, bottom 24 is composed of cartilage since it is desired to minimize bleeding and leave subchondral bone 22 undisturbed. Preferably, the placental membrane preparation substantially fills void 14.

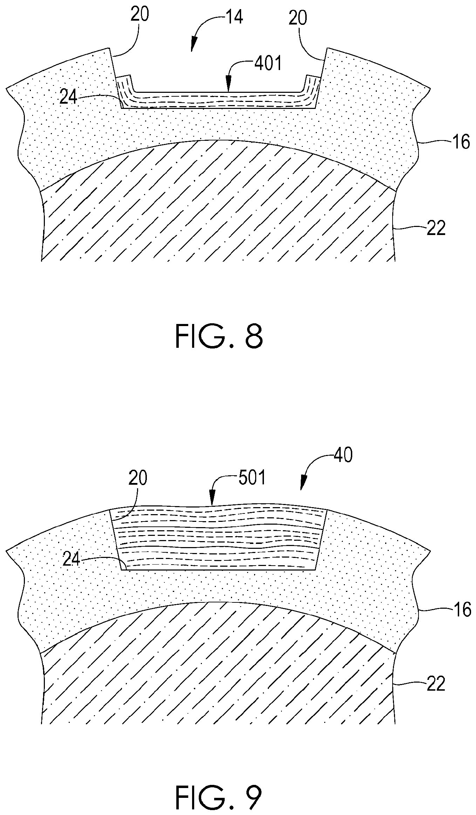

As an alternative to folded, although this is not part of the invention, implantable units 201 and 301, it is anticipated that the placental membrane preparations may be implanted into a skeletal joint in various unfolded orientations. For example, as depicted in FIG. 8, a placental membrane preparation may be presented as an implantable unit 401 having the amnion or epithelial side of unit 401 facing bottom 24 and the chorion side, impregnated with DBP, facing the upper opening. Implantable unit 401 is provided as a single, unfolded placental membrane layer covering bottom 24 of void 14, and optionally a portion of sidewall 20. Additionally, as depicted in FIG. 9, the placental membrane preparation may be presented as an implantable unit 501 composed of multiple, unfolded placental membrane layers stacked on top of one another. In this embodiment, the bottommost layer is preferably oriented with the amnion or epithelial side facing and covering bottom 24 with the topmost placental membrane layer oriented with its amnion or epithelial side facing the upper opening. The intermediate placental membrane layers can be oriented in whatever manner is deemed most advantageous. DBP is applied to the chorion side of one or more of the placental membrane lavers. Preferably, placental membrane layers of unit 501 are stacked within void 14 so that the upper surface of implantable unit 501 lies immediately below a plane formed by the upper opening.

Prior to implantation of the placental membrane preparation, the subchondral bone may inadvertently be perforated or abrasions formed. Perforations or abrasions in the subchondral bone or the calcified cartilage may induce bleeding and the formation of a fibrous clot in the defect, as well as the subsequent invasion of mesenchymal progenitor cells from the bone marrow to the site of the damaged cartilage. For this reason cartilage repair procedures currently in use such as microfracture intentionally perforate the subchondral bone in order to induce clotting and initiate repair. However, introduction of blood and/or mesenchymal progenitor cells from the bone marrow into the void may induce the formation of fibrocartilage in place of the desired hyaline cartilage. Accordingly, in the claimed technique the leakage of blood should be minimzed, and any blood clots that may form as a result of the blood leakage should be removed. Techniques for the removal of blood and blood clots are well-known to those skilled in the art. Such techniques may include, but are not limited to, for example, aspiration. Hemostatic agents including, but not limited to, bone wax may also be applied to the site of blood leakage, typically exposed subchondral bone.

Bone marrow may also be released from the subchondral bone, during or proximal to the implantation of the placental membrane preparation. The bone marrow may be removed using techniques well-known to those skilled in the art. Techniques include, but are not limited to, aspiration.

To further prevent formation of fibrocartilage cells from cells derived from the placental membrane sheets, the sheets are folded and arranged within the joint so that the largely impermeable, epithelial cell monolayer of the amnion forms the exterior of the implantable unit. In this way, blood that may collect within a void formed in a joint is separated or shielded from the interior of the implantable unit where chondrogenic growth and differentiation occurs in contact with cartilage on the lateral sides of the graft. By preventing the leakage of blood into the implantable unit, it is believed the mechanisms which cause fibrocartilage and osteoblast formation are substantially reduced or terminated.

### D. Example

The following example is presented to provide those of ordinary skill in the art with a comnlete disclosure and a description of how the compounds, compositions, and methods described and claimed herein are made and evaluated. The following examples are intended to be purely exemplary and are not intended to limit the scope of what the inventors regard as their invention. There are numerous variations and combinations of conditions, e.g., component concentrations, desired solvents, solvent mixtures, temperatures, pressures and other reaction ranges and conditions that can be used to optimize the product from the described process. Only reasonable and routine experimentation will be required to optimize such process conditions. As will be understood by those familiar with the art, the present invention may be embodied in other specific forms without departing from the spirit or essential characteristics thereof.

### 1. In vivo Cartilage Repair Trial

A study was performed for evaluating the use of human amniotic membrane mixed with demineralized bone to fill cartilage defects in a sheep model. It was hypothesized that this membrane would be able to fill these defects with chondrocyte-like cells and that the defects would be filled with hyaline cartilage.

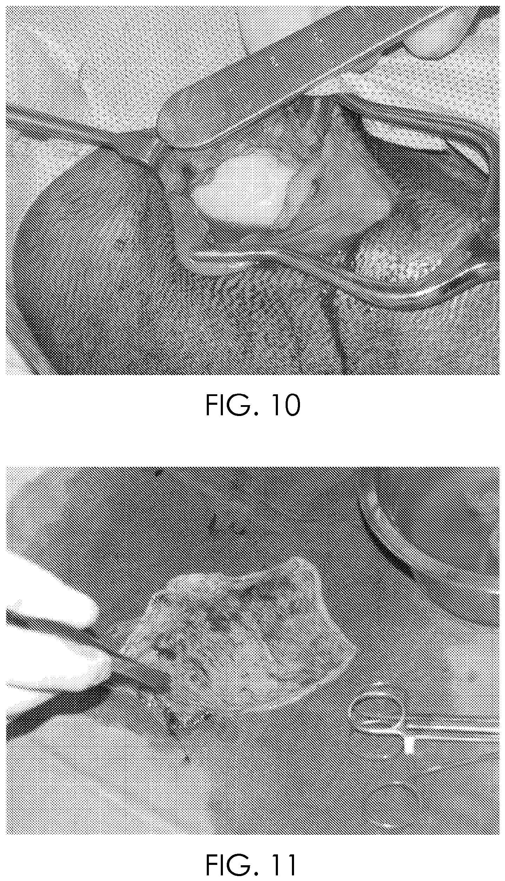

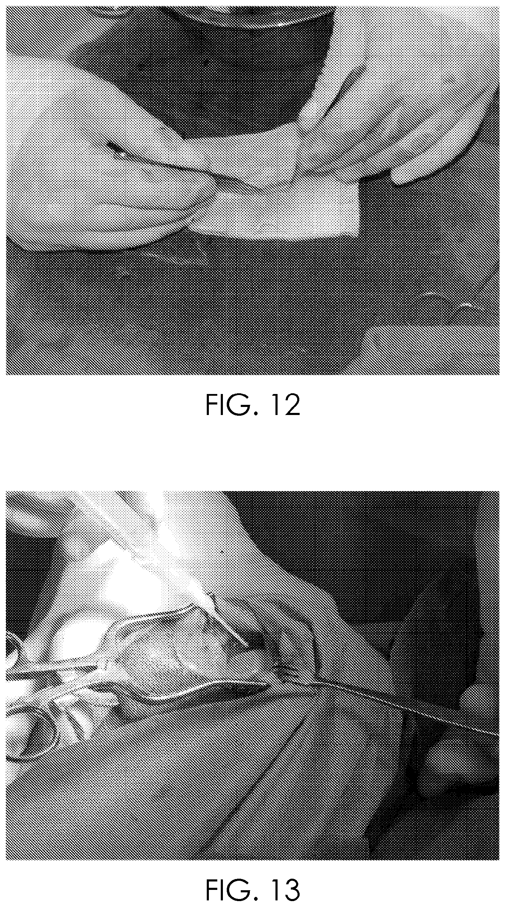

Method: Six adult sheep (less than three years old) where chosen for the study. Each sheep was anesthetized by a licensed veterinarian and one hind-quarter knee of each was sterilized and surgically exposed. As depicted in FIG. 10, two cartilage defects were created using curettes, one on the weight-bearing surface of the femoral condyle and one in the trochlear grove. The defects did not violate the subchondral bone. Three test sheep were used as control sheep and the cartilage defects were left unfilled. Three test sheep were chosen to receive human amniotic membrane implants. The amniotic membrane sheet was procured from a placenta and cut to fit the cartilage defect. As depicted in FIGS. 11 and 12, the membrane was folded into an implantable unit and arranged so that the cellular or epithelial layer faced the cartilage defect and the joint. Between the layers of the amnion membrane sheet, a small amount of demineralized bone was placed on a chorion side of the sheet. As depicted in FIG. 13, the amnion membrane implants were fixed to the cartilage defects on the femoral chondyles using micro bone anchors and fibrin glue. The amnion membrane implants were fixed to the trochlear defects using fibrin glue alone. The wounds were closed and the sheep were allowed to weight bear as tolerated. At six-months the sheep were sacrificed and the knees were harvested. Histological evaluation was made of the defects.

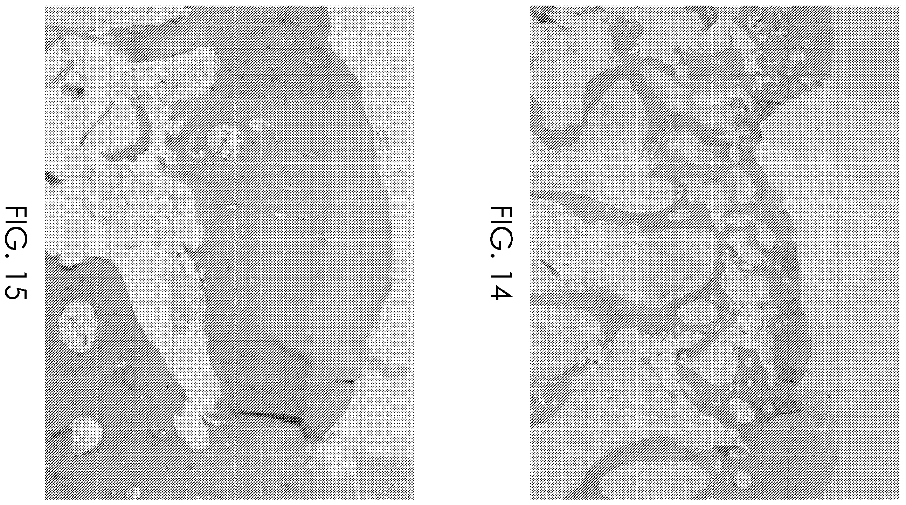

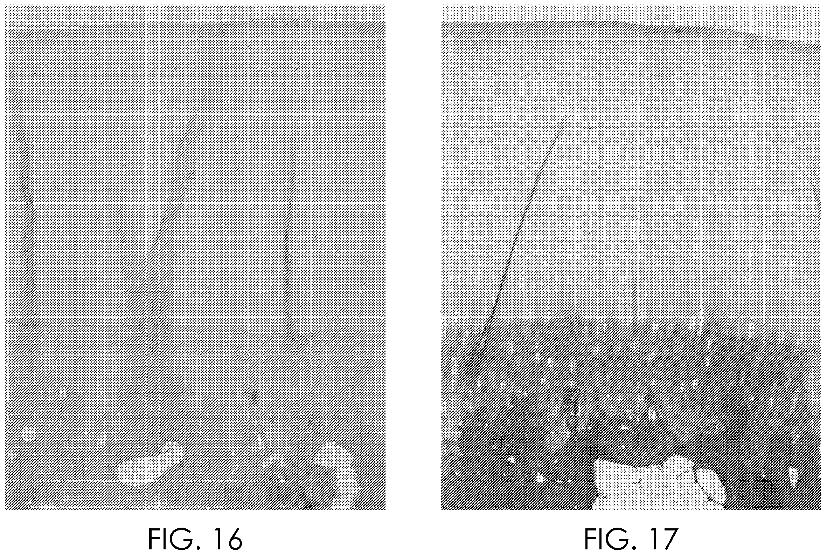

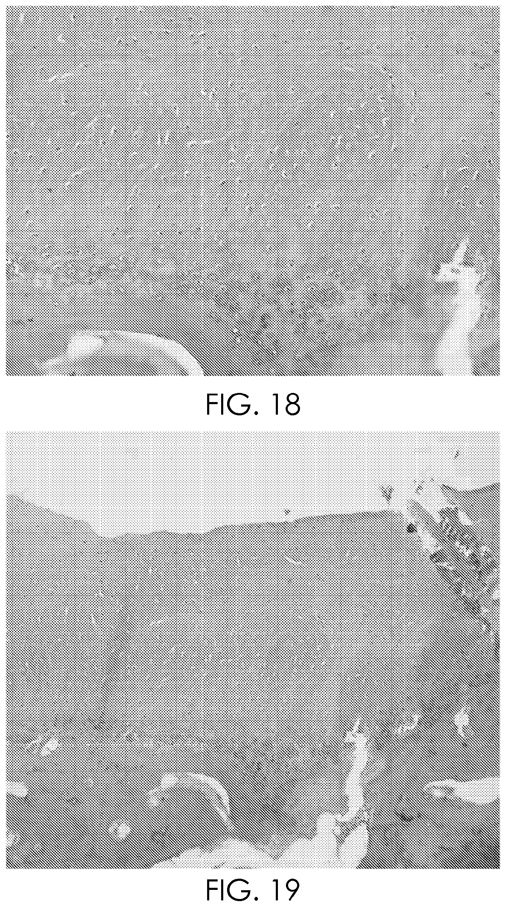

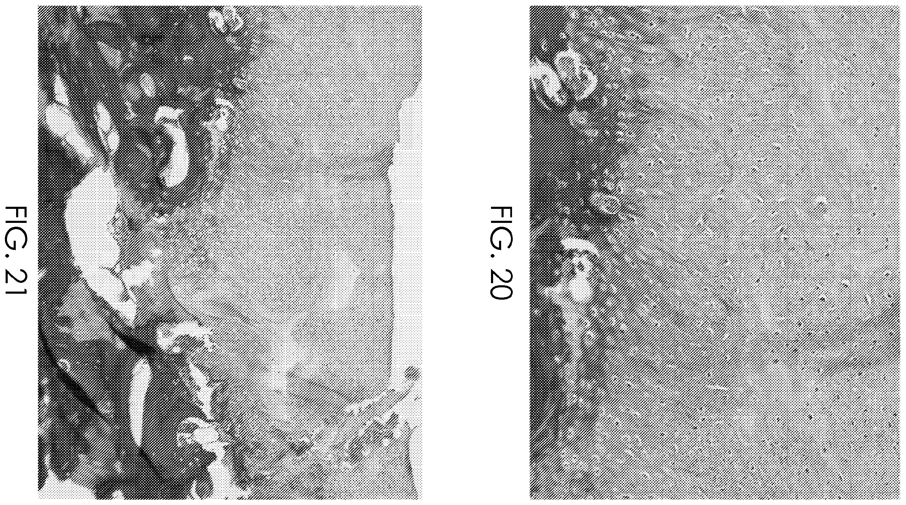

Results: Samples of the cartilage defects were examined histologically based on a simple, validated scoring system. The tests samples (depicted in FIGS. 18-21) were compared to the control samples (depicted in FIGS. 14 and 15) and the normal samples (depicted in FIGS. 16 and 17) taken from the sheep. Referring to the control sheep samples depicted in FIGS. 14 and 15, none of the cartilage defects in the control sheep filled in with hyaline cartilage or fibrocartilage. This is evident from the complete lack of tissue present in the voids created above the subchondral bone by the formation of the cartilage defects. The voids are represented in FIGS. 14 and 15 by the empty depressions defined by opposing vertically extending sidewalls which terminate at the upper ends thereof at the upper surface of the cartilage. In the test sheep in which the amnion membrane implants were placed, 50% of the defects appeared to retain the amnion membrane, which is consistent with other similar animal models. Referring to the test sheep samples depicted in FIGS. 18-21, the cartilage defects of the test sheep that retained their membranes had evidence of diffuse chondrocyte-like cell proliferation and showed a stromal matrix similar to hyaline cartilage. The graft samples of the test sheep defects showed 90% normal appearing cartilage compared with 40% normal in the control sheep. The grafts from the test sheep all scored a 3 on a 0-3 cartilage appearance scale compared with a 1.3 for the controls.

### References

1. Boo L, et al. A preliminary study of human amniotic membrane as a potential chondrocyte carrier, Malay Orthop J 3, 16-23 (2009).
2. Davis, JS, Skin transplantation with a review of 550 cases at the Johns Hopkins Hospital, John Hopkins Med J 15, 307 (1910).
3. Diaz-Prado SM, et al. Cell therapy and tissular engineering to regenerate articular cartilage, in BIOMEDICAL ENGINEERING, TRENDS, RESEARCH AND TECHNOLOGIES (Komorowska MA & Olsztynska-Janus S, eds.), pp. 193-216 (2011).
4. Diaz-Prado SM, et al. Potential use of the human amniotic membrane as a scaffold in human articular cartilage repair, Cell Tissue Bank 11, 183-195 (2010).
5. Hildner F, et al. State of the art and future perspectives of articular cartilage regeneration: a focus on adipose-derived stem cells and platelet-derived products, J Tissue Eng Regen Med 5, e36-e51 (2011).
6. Jin CZ, et al. Human amniotic membrane as a delivery matrix for articular cartilage repair, Tiss Eng 13, 693-702 (2007).
7. Kanthan SR, et al. The different preparations of human amniotic membrane (HAM) as a potential cell carrier for chondrocytes, Eur Cell Mater 20, 1 page (2010).
8. Krishnamurithy G, et al. Human amniotic membrane as a chondrocyte carrier vehicle/substrate: in vitro study, J Biomed Mater Res Part A 99A, 500-506 (2011).
9. Lindenmair A, et al. Osteogenic differentiation of intact human amniotic membrane, Biomaterials 31, 8659-8665 (2010).
10. Mermet I, et al. Use of amniotic membrane transplantation in the treatment of venous leg ulcers, Wound Repair and Regeneration 15, 459 (2007).
11. Moriya T, et al. Evaluation of reparative cartilage after autologous chondrocyte implantation for osteochondritis dissecans: histology, biochemistry, and MR imaging, J Orthop Sci 12, 265-273 (2007).
12. Niknejad H, et al. Properties of the amniotic membrane for potential use in tissue engineering, Eur Cell Mater 15, 88-99 (2008).
13. Wilshaw SP, et al. Production of an acellular amniotic membrane matrix for use in tissue engineering, Tiss Eng 12, 2117-2129 (2006).
14. Zhou S, et al. Demineralized bone promotes chondrocyte or osteoblast differentiation of human bone marrow stromal cells cultured in collagen sponges, Cell Tissue Bank 6, 33-44 (2005).
15. U.S. Patent No. 7,824,711.
16. U.S. Patent No. 6,063,094.
17. U.S. Patent No. 5,612,028.
18. U.S. Patent No. 5,004,468.
19. U.S. Patent No. 3,640,279.
20. U.S. Patent No. 3,472,228.
21. U.S. Patent No. 3,358,688.
22. U.S. Patent No. 20030187515
23. U.S. Patent No. 20040161419

## Claims

1. A placental membrane preparation comprising a demineralized bone powder (DBP) and a placental membrane sheet wherein the placental membrane sheet is folded to form an implantable unit; wherein the implantable unit has an exterior surface including an upper side and a lower side, at least about 90% of the exterior surface of the implantable unit presenting an exposed placental epithelial monolayer.

2. The preparation according to claim 1 wherein the placental membrane sheet excludes the chorion.

3. The preparation according to claim 2 wherein the DBP is applied to a stromal layer of the placental membrane sheet, a fibroblast layer of the placental membrane sheet or both.

4. The preparation according to claim 3 wherein the implantable unit includes a stromal layer located between two layers of epithelial cells.

5. The preparation according to claim 4 wherein the DBP is applied to the stromal layer.

6. The preparation according to claim 5 wherein the DBP is not applied to the two layers of epithelial cells.

7. The preparation according to claim 1 wherein the placental membrane sheet includes a plurality of chondrocytes derived from cells originating from the placental membrane sheet.

8. The preparation according to claim 7 wherein the placental membrane sheet is essentially free of osteoblasts.

9. The preparation according to claim 1 wherein the preparation is essentially free of *in vitro* cultured cells derived from sources other than the placental membrane sheet.

10. The preparation according to claim 1 wherein the preparation excludes growth factors derived from sources other than the placental membrane sheet and the DBP.

11. A method of making a placental membrane preparation comprising applying a DBP to a placental membrane sheet including a plurality of cells of the placental membrane sheet and inducing chondrogenic differentiation of the plurality of cells of the placental sheet, further comprising arranging the placental membrane sheet so that an amnion side of the placental membrane sheet faces outwardly in two substantially opposing directions and the chorion side of the placental membrane sheet faces inwardly.

12. The method according to claim 11 further comprising removing the chorion from the placental membrane sheet prior to applying the DBP thereto and applying the DBP to a stromal layer of the placental membrane sheet, a fibroblast layer of the placental membrane sheet or both.

13. The method according to claim 11 further comprising applying the DBP to the placental membrane sheet no more than about 36 hours prior to *in vivo* implantation of the preparation into a mammal.

14. The method according to claim 11 further comprising drying the placental membrane sheet, followed by rehydrating the placental membrane sheet, wherein the DBP is applied to the placental membrane sheet after the placental membrane sheet is rehydrated.

15. The method according to claim 11 further comprising drying the placental membrane sheet, followed by rehydrating the placental membrane sheet, wherein the DBP is applied to the placental membrane sheet while the placental membrane sheet is rehydrating.

16. The method according to claim 11 further comprising drying the placental membrane sheet, followed by rehydrating the placental membrane sheet, wherein the DBP is applied to the placental membrane sheet prior to rehydrating the placental membrane sheet.

17. The method according to claim 11 wherein the placental membrane sheet includes an amnion side having an exposed epithelial layer and a chorion side having a stromal layer.

18. The method according to claim 17 further comprising forming an implantable unit by arranging the placental membrane sheet to include a chorion side disposed between a first portion of the amnion side and a second portion of the amnion side.

19. The method according to claim 18 wherein the placental membrane sheet is essentially free of chondrocytes at the moment of implantation.

20. The method according to claim 11 further comprising removing the chorion from the placental membrane sheet, wherein the DBP is applied to a chorion side of the placental membrane sheet, followed by inducing chondrogenic differentiation of the plurality of amnionic mesenchymal cells of the placental membrane sheet.

21. The placental membrane preparation according to any of claims 1 to 10 for use in the treatment of chondral defects, osteoarthritis, traumatic injury, osteochondritis dessicans, pathological injury, age-related degeneration or other defects affecting skeletal joints, in particular cartilage.

22. The method according to claim 11 wherein the DBP is not applied to an amnion side of the placental membrane sheet.

23. The method according to claim 11 further comprising inducing chondrogenic differentiation of a plurality of cells of the placental membrane sheet in an essentially oxygen-free environment.

24. The method according to claim 11 further comprising DBP-induced chondrogenic differentiation of a plurality of cells of the placental membrane sheet with essentially no osteoblast formation.

25. The method according to claim 11 further comprising stacking a plurality of placental membrane sheets on the placental membrane sheet.

## Patentansprüche

1. Plazentamembranpräparat, das ein demineralisiertes Knochenpulver (DBP) und eine Plazentamembranschicht aufweist, wobei die Plazentamembranschicht gefaltet ist, um eine implantierbare Einheit zu bilden, wobei die implantierbare Einheit eine äußere Oberfläche aufweist, die einer obere Seite und eine untere Seite aufweist, wobei mindestens etwa 90 % der äußeren Oberfläche der implantierbaren Einheit eine freiliegende Plazentaepithel-Monoschicht aufweisen.

2. Präparat nach Anspruch 1, wobei die Plazentamembranschicht das Chorion ausschließt.

3. Präparat nach Anspruch 2, wobei das DBP auf eine stromale Schicht der Plazentamembranschicht, eine Fibroblastenschicht der Plazentamembranschicht oder beide aufgebracht sind.

4. Präparat nach Anspruch 3, wobei die implantierbare Einheit eine stromale Schicht enthält, die sich zwischen zwei Schichten von Epithelzellen befindet.

5. Präparat nach Anspruch 4, wobei das DBP auf die stromale Schicht aufgebracht ist.

6. Präparat nach Anspruch 5, wobei das DBP nicht auf die zwei Schichten von Epithelzellen aufgebracht ist.

7. Präparat nach Anspruch 1, wobei die Plazentamembranschicht eine Vielzahl von Chondrozyten enthält, die von Zellen stammen, die von der Plazentamembranschicht stammen.

8. Präparat nach Anspruch 7, wobei die Plazentamembranschicht im Wesentlichen frei von Osteoblasten ist.

9. Präparat nach Anspruch 1, wobei das Präparat im Wesentlichen frei von in vitro kultivierten Zellen ist, die aus anderen Quellen als der Plazentamembran stammen.

10. Präparat nach Anspruch 1, wobei das Präparat Wachstumsfaktoren ausschließt, die von anderen Quellen als der Plazentamembranschicht und dem DBP stammen.

11. Verfahren zur Herstellung eines Plazentamembranpräparats, wobei das Verfahren umfasst:
ein Aufbringen eines DBP auf eine Plazentamembranschicht, die eine Vielzahl von Zellen der Plazentamembranschicht aufweist, und
ein Induzieren einer chondrogenen Differenzierung der Vielzahl von Zellen der Plazentamembranschicht,
ferner ein Anordnen der Plazentamembranschicht auf eine solche Weise, dass eine Amnionseite der Plazentamembranschicht in zwei im Wesentlichen entgegen gesetzten Richtungen nach außen gerichtet ist und dass die Chorionseite der Plazentamembranschicht nach innen gerichtet ist.

12. Verfahren nach Anspruch 11, welches ferner umfasst:
ein Entfernen des Chorions von der Plazentamembranschicht vor dem Aufbringen des DBP und
ein Aufbringen des DBP auf eine stromale Schicht der Plazentamembranschicht, eine Fibroblastenschicht der Plazentamembranschicht oder beides.

13. Verfahren nach Anspruch 11, welches ferner umfasst:
ein Aufbringen des DBP auf die Plazentamembranschicht nicht länger als etwa 36 Stunden vor der In-vivo-Implantation des Präparats in ein Säugetier.

14. Verfahren nach Anspruch 11, welches ferner umfasst:
ein Trocknen der Plazentamembranschicht,
gefolgt von einem Rehydratisieren der Plazentamembranschicht, wobei das DBP auf die Plazentamembranschicht aufgebracht wird, nachdem die Plazentamembranschicht rehydriert worden ist.

15. Verfahren nach Anspruch 11, welches ferner umfasst:
ein Trocknen der Plazentamembranschicht,
gefolgt von einem Rehydratisieren der Plazentamembranschicht, wobei das DBP auf die Plazentamembranschicht aufgebracht wird, während die Plazentamembranschicht rehydriert wird.

16. Verfahren nach Anspruch 11, welches ferner umfasst:
ein Trocknen der Plazentamembranschicht,
gefolgt von einem Rehydratisieren der Plazentamembranschicht, wobei das DBP auf die Plazentamembranschicht vor der Rehydratisierung der Plazentamembranschicht aufgebracht wird.

17. Verfahren nach Anspruch 11, wobei die Plazentamembranschicht eine Amnionseite, die eine freiliegende Epithelschicht aufweist, und eine Chorionseite enthält, die eine stromale Schicht aufweist.

18. Verfahren nach Anspruch 17, welches ferner umfasst:
ein Bilden einer implantierbaren Einheit durch ein Anordnen der Plazentamembranschicht, um eine Chorionseite einzuschließen, die zwischen einem ersten Teil der Amnionseite und einem zweiten Teil der Amnionseite angeordnet ist.

19. Verfahren nach Anspruch 18, wobei die Plazentamembranschicht zum Zeitpunkt der Implantation im Wesentlichen frei von Chondrozyten ist.

20. Verfahren nach Anspruch 11, welches ferner umfasst:
ein Entfernen des Chorions von der Plazentamembranschicht, wobei das DBP auf eine Chorionseite der Plazentamembranschicht aufgebracht wird,
gefolgt von einem Induzieren einer chondrogenen Differenzierung der Vielzahl von amnionischen mesenchymalen Zellen der Plazentamembranschicht.

21. Plazentamembranpräparat nach einem der Ansprüche 1 bis 10 zur Behandlung von Chondraldefekten, Arthrose, traumatischen Verletzungen, Osteochondritis dessicans, pathologischen Verletzungen, altersbedingten Degenerationen oder anderen Defekten der Skelettgelenke, insbesondere Knorpel.

22. Verfahren nach Anspruch 11, wobei das DBP nicht auf eine Amnionseite der Plazentamembranschicht aufgebracht wird.

23. Verfahren nach Anspruch 11, welches ferner umfasst:
ein Induzieren einer chondrogenen Differenzierung einer Vielzahl von Zellen der Plazentamembranschicht in einer im Wesentlichen sauerstofffreien Umgebung.

24. Verfahren nach Anspruch 11, welches ferner umfasst:
eine durch ein DBP induzierte chondrogene Differenzierung einer Vielzahl an Zellen der Plazentamembranschicht mit im Wesentlichen keiner Osteoblastenbildung.

25. Verfahren nach Anspruch 11, welches ferner umfasst:
ein Stapeln einer Vielzahl von Plazentamembranschichten auf der Plazentamembranschicht.

## Revendications

1. Préparation de membrane placentaire comprenant une poudre d'os déminéralisé (POD) et une feuille de membrane placentaire dans laquelle la feuille de membrane placentaire est pliée pour former une unité implantable ; dans laquelle l'unité implantable a une surface extérieure comprenant une face supérieure et une face inférieure, au moins environ 90 % de la surface extérieure de l'unité implantable présentant une monocouche épithéliale placentaire exposée.

2. Préparation selon la revendication 1 dans laquelle la feuille de membrane placentaire exclut le chorion.

3. Préparation selon la revendication 2 dans laquelle la POD est appliquée sur une couche stromale de la feuille de membrane placentaire sur une couche de fibroblastes de la feuille de membrane placentaire ou sur les deux.

4. Préparation selon la revendication 3 dans laquelle l'unité implantable comporte une couche stromale située entre deux couches de cellules épithéliales.

5. Préparation selon la revendication 4 dans laquelle la POD est appliquée sur la couche stromale.

6. Préparation selon la revendication 5 dans laquelle la POD n'est pas appliquée sur les deux couches de cellules épithéliales.

7. Préparation selon la revendication 1 dans laquelle la feuille de membrane placentaire comporte une pluralité de chondrocytes dérivés de cellules provenant de la feuille de membrane placentaire.

8. Préparation selon la revendication 7 dans laquelle la feuille de membrane placentaire est essentiellement dépourvue d'ostéoblastes.

9. Préparation selon la revendication 1 dans laquelle la préparation est essentiellement dépourvue de cellules cultivées *in vitro* dérivées de sources autres que la feuille de membrane placentaire.

10. Préparation selon la revendication 1 dans laquelle la préparation exclut les facteurs de croissance dérivés de sources autres que la feuille de membrane placentaire et la POD.

11. Procédé de fabrication d'une préparation de membrane placentaire comprenant l'application d'une POD sur une feuille de membrane placentaire comportant une pluralité de cellules de la feuille de membrane placentaire et induisant une différenciation chondrogénique de la pluralité de cellules de la feuille placentaire, comprenant en outre l'agencement de la feuille de membrane placentaire de sorte qu'un côté amnios de la feuille de membrane placentaire est orienté vers l'extérieur dans deux directions sensiblement opposées et que le côté chorion de la feuille de membrane placentaire est orienté vers l'intérieur.

12. Procédé selon la revendication 11 comprenant en outre l'élimination du chorion de la feuille de membrane placentaire avant l'application de la POD sur celle-ci et l'application de la POD sur une couche stromale de la feuille de membrane placentaire sur une couche de fibroblastes de la feuille de membrane placentaire ou sur les deux.

13. Procédé selon la revendication 11 comprenant en outre l'application de la POD sur la feuille de membrane placentaire pas plus d'environ 36 heures avant l'implantation *in vivo* de la préparation chez un mammifère.

14. Procédé selon la revendication 11 comprenant en outre le séchage de la feuille de membrane placentaire, suivi de la réhydratation de la feuille de membrane placentaire, dans lequel la POD est appliquée sur la feuille de membrane placentaire après que la feuille de membrane placentaire est réhydratée.

15. Procédé selon la revendication 11 comprenant en outre le séchage de la feuille de membrane placentaire, suivi de la réhydratation de la feuille de membrane placentaire, dans lequel la POD est appliquée sur la feuille de membrane placentaire tandis que la feuille de membrane placentaire se réhydrate.

16. Procédé selon la revendication 11 comprenant en outre le séchage de la feuille de membrane placentaire, suivi de la réhydratation de la feuille de membrane placentaire, dans lequel la POD est appliquée sur la feuille de membrane placentaire avant la réhydratation de la feuille de membrane placentaire.

17. Procédé selon la revendication 11 dans lequel la feuille de membrane placentaire comprend un côté amnios ayant une couche épithéliale exposée et un côté chorion ayant une couche stromale.

18. Procédé selon la revendication 17 comprenant en outre la formation d'une unité implantable par agencement de la feuille de membrane placentaire pour comprendre un côté chorion disposé entre une première partie du côté amnios et une seconde partie du côté amnios.

19. Procédé selon la revendication 18 dans lequel la feuille de membrane placentaire est essentiellement dépourvue de chondrocytes au moment de l'implantation.

20. Procédé selon la revendication 11 comprenant en outre l'élimination du chorion de la feuille de membrane placentaire, dans lequel la POD est appliquée sur un côté chorion de la feuille de membrane placentaire, suivi de l'induction d'une différenciation chondrogénique de la pluralité de cellules mésenchymateuses amniotiques de la feuille de membrane placentaire.

21. Préparation de membrane placentaire selon l'une quelconque des revendications 1 à 10 pour une utilisation dans le traitement des défauts chondraux, de l'ostéoarthrite, d'une lésion traumatique, de l'ostéochondrite disséquante, d'une lésion pathologique, d'une dégénérescence liée à l'âge ou autres défauts affectant les articulations squelettiques, en particulier le cartilage.

22. Procédé selon la revendication 11 dans lequel la POD n'est pas appliquée sur un côté amnios de la feuille de membrane placentaire.

23. Procédé selon la revendication 11 comprenant en outre l'induction de la différenciation chondrogénique d'une pluralité de cellules de la feuille de membrane placentaire dans un environnement essentiellement dépourvu d'oxygène.

24. Procédé selon la revendication 11 comprenant en outre la différenciation chondrogénique induite par la POD d'une pluralité de cellules de la feuille de membrane placentaire avec essentiellement aucune formation d'ostéoblastes.

25. Procédé selon la revendication 11 comprenant en outre l'empilement d'une pluralité de feuilles de membrane placentaire sur la feuille de membrane placentaire.
